(19) Europäisches Patentamt
European Patent Office
Office européen des brevets

(11) Veröffentlichungsnummer: **0 161 595**
**A2**

# (12) EUROPÄISCHE PATENTANMELDUNG

(21) Anmeldenummer: **85105452.8**

(22) Anmeldetag: **04.05.85**

(51) Int. Cl.⁴: **A 61 K 7/08**, C 08 L 67/00

(30) Priorität: **12.05.84 DE 3417646**

(43) Veröffentlichungstag der Anmeldung: **21.11.85 Patentblatt 85/47**

(84) Benannte Vertragsstaaten: **AT BE CH DE FR GB IT LI LU NL SE**

(71) Anmelder: **Henkel Kommanditgesellschaft auf Aktien, Postfach 1100 Henkelstrasse 67, D-4000 Düsseldorf-Holthausen (DE)**

(72) Erfinder: **Höffkes, Horst, Dr., Carlo-Schmid-Strasse 113, D-4000 Düsseldorf-Hellerhof (DE)**
Erfinder: **Anzinger, Hermann, Dr., Jakob-Kneip-Strasse 146, D-4000 Düsseldorf (DE)**
Erfinder: **Worschech, Kurt, Dr., Alte Strasse 4, D-2854 Loxstedt (DE)**

## (54) Haarnachbehandlungsmittel.

(57) Haarnachbehandlungsmittel in Form einer wäßrigen Emulsion von Fettstoffen und mit einem Gehalt von kationischen Tensiden enthalten als Fettstoffe ein Gemisch aus gesättigten $C_{12}$-$C_{22}$-Fettalkoholen und Komplexestern aus aliphatischen Polyolen, Dicarbonsäuren und aliphatischen Monocarbonsäuren. Das Verhältnis von Fettalkoholen zu Komplexestern kann von 1:9 bis 9:1 betragen. Die Haarbehandlungsmittel enthalten bevorzugt 1-10 Gew.% solcher Fettstoffe, weisen eine verbesserte Viskositätsstabilität auf und verbessern die Naßkämmbarkeit des Haares.

EP 0 161 595 A2

Henkelstraße 67
4000 Düsseldorf, den 9.5.1984

HENKEL KGaA
ZR-FE/Patente
Dr. JG/Br

Patentanmeldung

D 6945 EP

"Haarnachbehandlungsmittel"

Die Erfindung betrifft Haarnachbehandlungsmittel in Form einer Emulsion von Fettstoffen in Wasser und mit einem Gehalt an kationischen Tensiden.

Das Haupthaar weist nach dem Waschen mit Shampoos, Duschbädern und Badepräparaten auf Basis von synthetischen Tensiden, aber auch nach kosmetischen Behandlungen wie Färben, Bleichen und Verformen oft einen kosmetisch unbefriedigenden Zustand auf. Es ist im nassen Zustand schlecht zu kämmen und zeigt im trockenen Zustand wenig Glanz, Halt und Fülle. Vor allem neigt es zur statischen Aufladung, wodurch das "Fliegen" des frisch gewaschenen Haares verursacht wird.

Es ist bekannt, diesen Übelstand durch die Anwendung von Haarnachbehandlungsmitteln abzustellen. Solche Haarspülmittel enthalten als Wirkstoffe mit substantiver, antistatischer Wirkung kationische Tenside, bevorzugt vom Typ der quartären Ammoniumverbindungen. Geeignete kationische Tenside werden z.B. im "Handbuch der Kosmetika und Riechstoffe" von Hugo Janistyn, III. Band, 2. Auflage (1973) auf Seite 419-420 genannt. Zur Behebung der statischen Aufladbarkeit genügt es, wässrige Lösungen solcher kationischer Tenside auf das Haar aufzubringen. Es ist jedoch erwünscht, daß solche Haarspülmittel einen zusätzlichen avivierenden Effekt - zur Verbesserung von Glanz und Fülle des Haares - aufweisen und daß sie ein cremiges, gehaltvolles Aussehen haben. Dies läßt sich dadurch erreichen, daß

...

Fettstoffe, z.B. Fettalkohole, Fettsäureglyceride, Fettsäureester oder Paraffine in emulgierter Form in die Haarspülmittel eingesetzt werden. Die gebräuchlichsten Fettkörper für diesen Zweck sind Fettalkohole, insbesondere Cetylalkohol und Stearylalkohol und Fettsäurepartialglyceride, z.B. Mischungen aus Glycerinmono- und difettsäureestern.

Die emulsionsförmigen Haarnachspülmittel mit einem Gehalt an Fettalkoholen wirken aber wenig ansprechend. Sie sehen durchscheinend aus und sind wenig cremig. Bei höheren Konzentrationen an Fettalkohol nimmt die Fließfähigkeit stark ab und die Formulierungen werden gelartig.

Ein weiterer Nachteil solcher bekannter Formulierungen besteht darin, daß die Viskosität der Emulsionen im Laufe von Stunden und Tagen nach der Herstellung stark zunimmt, so daß ein gezielter Konsistenzaufbau sehr erschwert ist.

Durch teilweisen oder vollständigen Ersatz der Fettalkohole durch Fettsäurepartialglyceride wie Glycerinmono/distearat läßt sich dieser Nachteil etwas verringern, aber die so erhaltenen Produkte weisen eine unzureichende Verbesserung der Naßkämmbarkeit auf.

Es bestand daher die Aufgabe, emulsionsförmige Haarnachbehandlungsmittel zu finden, die sowohl bezüglich des Aussehens und der Viskositätsstabilität als auch bezüglich der Kämmbarkeitsverbesserung die bestehenden Nachteile nicht aufweisen.

Diese Aufgabe wurde erfindungsgemäß gelöst durch Haarnachbehandlungsmittel in Form einer wässrigen Emulsion

...

von Fettstoffen und mit einem Gehalt an kationischen Tensiden, dadurch gekennzeichnet, daß die Fettstoffe im wesentlichen ein Gemisch aus gesättigten Fettalkoholen mit 12 bis 22 C-Atomen und Komplexestern im Gewichtsverhältnis 9 : 1 bis 1 : 9, bevorzugt 1 : 1 darstellen, wobei die Komplexester aus

n Mol eines aliphatischen Polyols mit 3 bis 12 C-Atomen und m Hydroxylgruppen,

(n-1) Mol einer Dicarbonsäure mit 4 bis 22 C-Atomen und

2 bis $[n \cdot m - 2\,(n-1)]$ Mol einer aliphatischen Monocarbonsäure mit 8 bis 22 C-Atomen

durch Veresterung erhältlich sind und wobei n eine ganze Zahl von 2 bis 11 und m, die Funktionalität des Polyols, wenigstens 3 ist.

Komplexester der genannten Art sind an sich bekannte Verbindungen oder nach bekannten Verfahren herstellbar. Flüssige Komplexester dieser Art sind z.B. aus US-PS 3,000,917 bekannt. Feste Komplexester dieses Typs werden in DE-PS 19 07 768 beschrieben. In diesen Druckschriften werden auch Verfahren zur Herstellung solcher Komplexester (oder Mischester) angegeben.

Als Polyole zur Herstellung geeigneter Komplexester kommen z.B. Glycerin, Pentaerythrit, Hexantriol, Trimethylolpropan, Erythrit, Xylit, Sorbit oder Dimere von Trimethylolpropan, Pentaerythrit oder Glycerin in Frage. Bevorzugt geeignet sind Glycerin und Pentaerythrit. Als Dicarbonsäuren können z.B. Bernsteinsäure, Glutarsäure, Adipinsäure, Pimelinsäure, Korksäure, Azelainsäure, Sebazinsäure, Nonandicarbonsäure, Undecandicarbonsäure, Maleinsäure, Fumarsäure, Cyclopropandi-

...

carbonsäure, Cyclobutandicarbonsäure, Camphersäure, Hexahydrophthalsäuren, aber auch Phthalsäure, Terephthalsäure, Isophthalsäure, Diphenyl-O,O'-Dicarbonsäure oder Mischungen der genannten Dicarbonsäuren eingesetzt werden. Bevorzugt geeignete Komplexester werden mit linearen $\alpha$-$\omega$-Dicarbonsäuren mit 6 bis 10 C-Atomen erhalten.

Als Beispiele für geeignete aliphatische Monocarbonsäuren sind zu nennen: Caprylsäure, Caprinsäure, Laurinsäure, Myristinsäure, Palmitinsäure, Stearinsäure ,Ölsäure, Linolsäure, Arachinsäure, Behensäure und Erucasäure sowie technische Gemische der genannten Monocarbonsäuren. Obwohl solche linearen Fettsäuren mit 8 bis 22 C-Atomen bevorzugt sind, lassen sich geeignete Komplexester auch unter Verwendung von verzweigten Monocarbonsäuren wie z.B. 2-Ethylhexansäure, Isostearinsäure, 2-Hexyl-decansäure und 2-Octyl-dodecansäure herstellen.

Die Herstellung der Komplexester kann in einer Stufe durch Veresterung der Reaktionskomponenten in Gegenwart eines Veresterungskatalysators wie z.B. p-Toluolsulfonsäure, Natriumhydrogensulfat, sauren Ionenaustauschern, Zinnschliff oder Tetraisopropyltitanat durchgeführt werden. Es kann aber auch in zwei Stufen - zuerst das Polyol mit der Monocarbonsäure, dann dieses Veresterungsprodukt mit der Dicarbonsäure-, verestert werden.

Die erhaltenen Komplexester können bei Normaltemperatur flüssig sein oder wachsartige Konsistenz mit Schmelzpunkten bis zu 60 bis 80 °C haben. Wenn weniger als $[n \cdot m - 2(n - 1)]$ Mol der Monocarbonsäure zur Veresterung eingesetzt werden, enthält der Komplexester

...

freie Hydroxylgruppen, d.h. das Veresterungsprodukt weist eine entsprechend hohe Hydroxylzahl auf. Alle Komplexester im Rahmen des erfindungsgemäßen Bereiches bewirken in den erfindungsgemäßen Haarnachbehandlungsmitteln eine merkliche Verbesserung der Viskositätsstabilität und verbessern das cremige, gehaltvolle Aussehen der Emulsion. Auch die Naßkämmbarkeit des Haares und der Glanz des trockenen Haares werden spürbar verbessert. Es hat sich jedoch gezeigt, daß, wenn man noch fließbare Emulsionen mit relativ hohem Gehalt an emulgierten Fettstoffen oder Emulsionen mit niedriger Viskosität erhalten will, bevorzugt wachsartige Komplexester und solche mit niedriger Hydroxylzahl einsetzt. Will man andererseits Emulsionen herstellen, die bei geringerem Fettstoffgehalt bereits relativ dickflüssig sind, so setzt man bevorzugt flüssige Komplexester und solche mit freien Hydroxylgruppen ein. Eine weitere Möglichkeit, die Viskosität und das Aussehen der erfindungsgemäßen Haarnachbehandlungsemulsionen zu beeinflussen ist die Wahl des Mischungsverhältnisses aus gesättigten Fettalkoholen und Komplexestern in dem Bereich von 1 : 9 bis 9 : 1. Ein Mischungsverhältnis von ca. 1 : 1 hat sich jedoch als besonders geeignet erwiesen, da hierbei die günstigen Eigenschaften des Fettalkohols, insbesondere die leichte Emulgierbarkeit und die gute Emulsionsstabilität, noch ausreichend zur Geltung kommen. Haarnachbehandlungsemulsionen, die keinen oder zu wenig gesättigten Fettalkohol enthalten, können in Bezug auf die Lagerstabilität der Emulsion problematisch sein.

Die erfindungsgemäßen Haarnachbehandlungsemulsionen enthalten die Fettstoffe - also im wesentlichen die Mischung aus gesättigtem Fettalkohol und Komplexester -

...

bevorzugt in Mengen von 1 bis 10 Gewichtsprozent der Emulsion. Bevorzugt geeignete Fettalkohole sind Cetylalkohol und Stearylalkohol oder Mischungen dieser Alkohole. Bevorzugt geeignete Komplexester werden erhalten aus Glycerin oder Pentaerythrit, linearen $\alpha$-$\omega$-Dicarbonsäuren mit 6 bis 10 C-Atomen und linearen Fettsäuren mit 8 bis 22 C-Atomen. Besonders geeignete Beispiele solcher Komplexester sind z.B. die Veresterungsprodukte aus

- 7 Mol Pentaerythrit, 6 Mol Adipinsäure und 16 Mol Stearinsäure (Tropfpunkt 55 bis 57 °C, OH-Zahl unter 12)

- 5 Mol Pentaerythrit, 4 Mol Adipinsäure und 12 Mol Ölsäure (Stockpunkt unter -20 °C, OH-Zahl unter 12)

- 8 Mol Glycerin, 7 Mol Adipinsäure und 10 Mol Stearinsäure (Tropfpunkt 48 bis 50 °C, OH-Zahl unter 12)

- 2 Mol Glycerin, 1 Mol Adipinsäure und 2 Mol Stearinsäure (Tropfpunkt 50 bis 51 °C, OH-Zahl ca. 130)

- 2 Mol Glycerin, 1 Mol Adipinsäure und 2 Mol Ölsäure (Stockpunkt unter -10 °C, OH-Zahl ca. 120)

- 2 Mol Glycerin, 1 Mol Adipinsäure und 2 Mol Capryl-/Caprinsäure-Gemisch (1:1) (Stockpunkt ca. -35 °C, OH-Zahl ca. 187)

- 2 Mol Glycerin, 1 Mol Adipinsäure und 4 Mol Capryl/Caprinsäure-Gemisch (1:1) (Stockpunkt ca. -40 °C, OH-Zahl unter 10).

Daneben können die Fettstoffe der Haarnachbehandlungsemulsion noch geringe Mengen anderer Fettkomponenten

...

- z.B. Paraffine, Fettsäurepartialglyceride, kosmetische Ölkomponente (z.B. Isopropylstearat, Fettsäuretriglyceride) enthalten. Deren Menge sollte aber nicht mehr als 10 Gewichtsprozent der Fettstoffe ausmachen.

Die erfindungsgemäßen Haarnachbehandlungsemulsionen enthalten daneben die in solchen Emulsionen üblichen Zusätze und Hilfsmittel wie z.B. kationische Tenside, bevorzugt vom Typ der quartären Ammoniumverbindungen in Mengen von 0,5 bis 5,0 Gewichtsprozent. Weitere übliche Zusatzmittel in kleineren Mengen sind z.B. niedere Polyole (z.B. 1.2-Propylenglycol, Glycerin), wasserlösliche Polysaccharidderivate (z.B. Hydroxyethylcellulose, Methyl-hydroxypropyl-cellulose, Hydroxyethylstärke, Hydroxypropylguar), Konservierungsstoffe, Duftstoffe, Farbstoffe, Lichtschutzmittel sowie haarkosmetische Wirkstoffe wie z.B. Vitamine, Pflanzenextrakte, Balsame, Antischuppenwirkstoffe (z.B. Zn- oder Mg-Pyridinthion), oder Sebostatika.

Als kationische Tenside mit antistatischer Wirkung sind besonders quartäre Ammoniumverbindungen der allgemeinen Formel

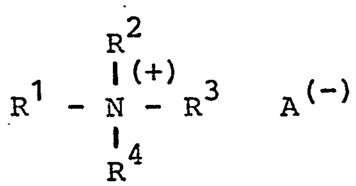

geeignet, in der $R^1$ eine vorzugsweise lineare Alkylgruppe mit 8 bis 18 C-Atomen, $R^2$ und $R^3$ Alkylgruppen mit 1 bis 4 C-Atomen oder Hydroxyalkylgruppen mit 2 bis 4 C-Atomen, $R^4$ eine Benzylgruppe oder eine Gruppe $R^2$ oder $R^3$ und $A^{(-)}$ ein wasserlösliches Anion, bevorzugt aus der Gruppe $Cl^{(-)}$, $Br^{(-)}$, $R^5\ SO_4^{(-)}$ darstellt, wobei $R^5$ Wasserstoff, eine Methyl- oder Ethylgruppe sein kann. Solche quartären Ammoniumverbindungen werden in die erfindungsgemäßen Haarnachbehandlungsemulsionen in Mengen

...

von 0,1 bis 2,0 Gewichtsprozent der Emulsion eingesetzt. Beispiele solcher bevorzugt geeigneter kationischer Tenside sind z.B. Cetyltrimethylammoniumchlorid, Lauryldimethylbenzylammoniumchlorid, Stearyl-pentaoxyethylammoniumchlorid. Die kationischen Tenside dienen sowohl als antistatische Wirkstoffe zur Verringerung der statischen Aufladbarkeit des Haares als auch zur Emulgierung der Fettstoffe. Die folgenden Beispiele sollen die Wirkung der erfindungsgemäßen Haarnachbehandlungsmittel näher erläutern.

...

## Beispiele

Anwendungstechnische Vergleiche:

Es wurden Haarnachbehandlungs-Emulsionen der in Tabelle I angegebenen Zusammensetzungen hergestellt:

Tabelle I

| Bestandteile (Gew.-%) | 1 | 2 | Vergleich 3 |
|---|---|---|---|
| Cetyl-Stearylalkohol (1 : 1) | 2,5 | 2,5 | 5,0 |
| Komplexester GAO (2 : 1 : 2) | 2,5 | - | - |
| Komplexester PAS (7 : 6 : 16) | - | 2,5 | - |
| Cetyltrimethylammoniumchlorid | 0,5 | 0,5 | 0,5 |
| Wasser | 94,5 | 94,5 | 94,5 |
| Viskosität der Emulsion [mPa s] | | | |
| nach 24 Stunden (23 °C) | 5250 | 1100 | 2450 |
| nach 3 Tagen (23 °C) | 5800 | 1450 | 4700 |
| nach 4 Tagen (23 °C) | 5500 | 1400 | 4800 |

Herstellung der Haarbehandlungsemulsionen

Die Fettkomponenten (Cetyl-Stearylalkohol, Komplexester) wurden gemeinsam aufgeschmolzen und die Schmelze auf 80 °C erwärmt. Das Cetyltrimethylammoniumchlorid wurde im Wasser gelöst und die Lösung ebenfalls auf 80 bis 90 °C erwärmt. Dann wurde unter intensivem Rühren die wässrige Lösung in die Schmelze der Fettkomponenten einemulgiert. Die fertige Emulsion wurde innerhalb von 1 bis 2 Stunden auf Raumtemperatur (23 °C) abgekühlt.

...

Als Komplexester wurden folgende Produkte eingesetzt:

GAO (2:1:2) : Ein Veresterungsprodukt aus 2 Mol Glycerin, 1 Mol Adipinsäure und 2 Mol Ölsäure mit folgenden technischen Daten:

| | | |
|---|---|---|
| Trübungspunkt | : | ca. + 5 °C |
| Stockpunkt | : | unter - 10 °C |
| Säurezahl | : | unter 1 |
| Verseifungszahl | : | ca. 280 |
| Jodzahl | : | ca. 66 |
| Hydroxylzahl | : | ca. 120 |

PAS (7:6:16) : Ein Veresterungsprodukt aus 7 Mol Pentaerythrit, 6 Mol Adipinsäure und 16 Mol Stearinsäure mit folgenden technischen Daten:

| | | |
|---|---|---|
| Tropfpunkt | : | 55 - 57 °C |
| Säurezahl | : | unter 15 |
| Verseifungszahl | : | ca. 277 |
| Jodzahl | : | unter 2 |
| Hydroxylzahl | : | unter 12 |

Die Ausbildung der Viskosität der Haarbehandlungs-Emulsionen wurde mit Hilfe eines Brookfield-Rotationsviskosimeters (Type RVF, 20 UPM, Spindel 4) bei 23 °C nach 1, 3 und 4 Tagen überprüft (Tabelle I).

Die erfindungsgemäßen Emulsionen (Rezepturen 1 und 2) zeigten ein weißes, cremiges, gehaltvolles Aussehen und eine hohe Viskositätskonstanz.

Die Emulsion ohne Komplexester (Rezeptur 3) zeigte ein wenig ansprechendes Aussehen. Die Viskosität stieg nach 3 bis 4 Tagen stark an.

...

Die mit den erfindungsgemäßen Haarnachbehandlungs-Emulsionen (Rezeptur 1 und 2) behandelten Haare zeigten eine bessere Kämmbarkeit (Naßkämmbarkeit) als die mit Rezeptur 3 behandelten Haare.

...

## Patentansprüche

1. Haarnachbehandlungsmittel in Form einer wässrigen Emulsion von Fettstoffen und mit einem Gehalt an kationischen Tensiden, dadurch gekennzeichnet, daß die Fettstoffe im wesentlichen ein Gemisch aus gesättigten Fettalkoholen mit 12 bis 22 C-Atomen und Komplexestern im Gewichtsverhältnis 9 : 1 bis 1 : 9, bevorzugt 1 : 1 darstellen, wobei die Komplexester aus

   n Mol eines aliphatischen Polyols mit 3 bis 12 C-Atomen und m Hydroxylgruppen

   (n-1) Mol einer Dicarbonsäure mit 4 bis 22 C-Atomen und

   2 bis $[n \cdot m - 2(n-1)]$ Mol einer aliphatischen Monocarbonsäure mit 8 bis 22 C-Atomen

   durch Veresterung erhältlich sind und wobei n eine ganze Zahl von 2 bis 11 und m, die Funktionalität des Polyols, wenigstens 3 ist.

2. Haarnachbehandlungsmittel nach Anspruch 1, dadurch gekennzeichnet, daß die Fettstoffe in einer Menge von 1 bis 10 Gewichtsprozent enthalten sind und im wesentlichen aus einem Gemisch aus Cetylalkohol und/oder Stearylalkohol und einem Komplexester aus Glycerin oder Pentaerythrit, einer linearen $\alpha$-$\omega$-Dicarbonsäure mit 6 bis 10 C-Atomen und einer linearen Fettsäure mit 8 bis 22 C-Atomen bestehen.

...

3. Haarnachbehandlungsmittel nach Anspruch 1 oder 2, dadurch gekennzeichnet, daß als kationisches Tensid eine quartäre Ammoniumverbindung der allgemeinen Formel

$$\begin{matrix} & & R^2 & & \\ & & \mid^{(+)} & & \\ R^1 & - & N & - & R^3 \\ & & \mid & & \\ & & R^4 & & \end{matrix} \qquad A^{(-)}$$

in der $R^1$ eine vorzugsweise lineare Alkylgruppe mit 8 bis 18 C-Atomen, $R^2$ und $R^3$ Alkylgruppen mit 1 bis 4 C-Atomen oder Hydroxyalkylgruppen mit 2 bis 4 C-Atomen, $R^4$ eine Benzylgruppe oder eine Gruppe $R^2$ oder $R^3$ und $A^{(-)}$ ein wasserlösliches Anion, bevorzugt aus der Gruppe $Cl^{(-)}$, $Br^{(-)}$, $R^5\ SO_4^{(-)}$ darstellt, wobei $R^5$ Wasserstoff, eine Methyl- oder Ethylgruppe sein kann, in einer Menge von 0,1 bis 2,0 Gewichtsprozent enthalten ist.